# EUROPÄISCHE PATENTSCHRIFT

(11) **EP 3 504 182 B1**
(45) Veröffentlichungstag und Bekanntmachung des Hinweises auf die Patenterteilung: **08.11.2023**
(21) Anmeldenummer: 17757709.5
(22) Anmeldetag: 11.08.2017
(51) Int. Cl.: C08G 64/30, B01D 3/00, B01D 3/34

(54) **VERFAHREN ZUR HERSTELLUNG EINES POLYCARBONATS UNTER EINSATZ EINER STRIPPVORRICHTUNG**
METHOD FOR PRODUCING A POLYCARBONATE USING A STRIPPING DEVICE
PROCÉDÉ POUR PRODUIRE UN POLYCARBONATE EN EMPLOYANT UN DISPOSITIF DE STRIPPAGE

(30) Priorität: 29.08.2016 DE 102016116078
(43) Veröffentlichungstag der Anmeldung: 03.07.2019
(73) Patentinhaber: EPC Engineering & Technologies GmbH, 99310 Arnstadt (DE)
(72) Erfinder: STRENG, Michael, 63477 Maintal (DE); KLOOS, Dieter, 63739 Aschaffenburg (DE); BRACHT, Malte, 63457 Hanau (DE)
(74) Vertreter: Meissner Bolte Partnerschaft mbB
(86) Internationale Anmeldenummer: PCT/EP2017/070430
(87) Internationale Veröffentlichungsnummer: WO 2018/041602

(56) Entgegenhaltungen:
- WO-A1-2016/001164
- DE-A1- 2 439 552
- DE-A1- 19 903 459
- US-A1- 2011 105 708
- US-B1- 6 545 187
- US-B2- 8 022 240

## Beschreibung

Die vorliegende Erfindung betrifft ein Verfahren zur Herstellung eines Polycarbonats durch Reaktion eines oder mehrerer Diarylcarbonate mit einem oder mehreren aromatischen Hydroxyverbindungen, wobei Diarylcarbonat und aromatische Hydroxyverbindungen zunächst in einer Mischvorrichtung gemischt und als Rohstoffgemisch in einem Rohstoffgemisch-Behälter gelangt um anschließend in einem oder mehreren Reaktoren bei reduziertem Druck und erhöhter Temperatur zu Polycarbonat zu reagieren, wobei das Gemisch aus Diarylcarbonat und aromatische Hydroxyverbindung vor Eintritt in den Rohstoffgemischbehälter einer Reinigung unterzogen wird.

Aromatisches Polycarbonat ist aufgrund seiner guten mechanischen und optischen Eigenschaften ein wirtschaftlich interessanter Kunststoff mit zahlreichen Anwendungen. Anwendungen finden sich zum Beispiel in der Automobilbranche, in der Medizintechnik sowie der Verpackungsindustrie und vielen weiteren mehr.

Die Herstellung von aromatischem Polycarbonat in industriellem Maßstab wird heutzutage entweder durch ein Oberflächenverfahren oder durch ein Schmelzverfahren realisiert.

Im sog. Oberflächenverfahren wird ein aromatisches Hydroxyl mit Phosgen, einer wässrigen Lauge und einem Lösungsmittel durch Zugabe von einem Katalysator zu Polycarbonat umgesetzt. Das in Lösung anfallende Polycarbonat wird anschließend in mehreren Reinigungsschritten konzentriert.

Im Schmelzeverfahren wird mindestens eine aromatische Hydroxyverbindung, bevorzugt Bisphenol A, mit mindestens einem Diarylcarbonat, bevorzugt Diphenylcarbonat, zu Polycarbonat zur Reaktion gebracht. Hierfür werden die flüssigen Rohstoffströme vermischt und unter Zugabe von Katalysatoren bei erhöhter Temperatur und reduziertem Druck zu Polycarbonat umgesetzt.

Die Herstellung von Polycarbonaten nach dem genannten Schmelzumesterungsverfahren ist bekannt und beispielsweise beschrieben in "schnell", Chemistry and Physics of Polycarbonats, Polymer Reviews, Vol. 9, Interscience Publishers, New York, London, Sydney 1964, in D.C. Prevorsek, B.T. Debona and Y. Kersten, Corporate Research Center, Allied Chemical Corporation, Moristown, New Jersey 07960, "Synthesis of Poly(ester)carbonate Copolymers" in Journal of Polymer Science, Polymer Chemistry Edition, Vol. 19, 75-90 (1980), in D. Freitag, U. Grigo, P.R. Müller, N. Nouvertne, BAYER AG, "Polycarbonates" in Encyclopedia of Polymere Science and Engineering, Vol. 11, Second Edition, 1988, Seiten 648-718 und schließlich in Dres. U. Grigo, K. Kircher und P.R. Müller "Polycarbonate" in Becker/Braun, Kunststoff-Handbuch, Band 3/1, Polycarbonate, Polyacetale, Polyester, Celluloseester, Carl Hanser Verlag München, Wien 1992, Seiten 117-299.

Die bei der Herstellung von Polycarbonaten aus Bisphenolen und Diarylcarbonaten ablaufende Reaktion wird durch die folgende Gleichung wiedergegeben:

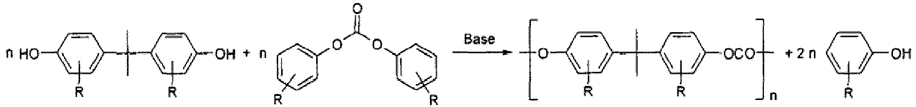

Aufgrund der geringeren Abwassermenge und dem nicht Bedarf von toxischem Phosgen wird das Schmelzeverfahren heutzutage vermehrt angewendet.

Es ist allerdings für das Schmelzeverfahren von größter Bedeutung hoch reine Rohstoffe zu verwenden, um ein qualitativ hochwertiges Polycarbonate herstellen zu können. Zu den Verunreinigungen gehören zum einen nicht abgetrennte Stoffe aus dem Herstellungsverfahren der Rohstoffe, wie zum Beispiel Phenol, Aceton, Wasser, Katalysatorreste, Phenyl Salicylate, Methoxyphenylbenzoate, Phenyl methyl carbonate, Dimethylcarbonate. Zum anderen können Verunreinigungen neu entstehen aufgrund von Zerfallsreaktionen der Rohstoffe.

Die Anzahl der möglichen Verunreinigungen hat zur Folge, dass es auch eine Vielzahl von Reaktionspfaden gibt über die Strukturänderungen des Produktes entstehen können. Hierzu zählen vor allem diejenigen, die die Kettenstruktur und oder die Kettenlänge beeinflussen. Im Gegensatz zu Verfärbungen die mittels Färbemittel korrigiert werden können, haben Änderungen der Kettenstruktur und oder der Kettenlänge veränderte bzw. verschlechterte rheologische Eigenschaften zur Folge, die hinterher nicht mehr korrigiert werden können und im schlimmsten Fall für den Hersteller einen kompletten Produktionsausfall bedeuten.

Es ist allgemein bekannt das bestimmte Verunreinigungen Bestandteil der Polymerkette werden können und dessen lineare Struktur durch Verzweigungen und Quervernetzungen verändern. Die Abweichungen führen dann zu unerwünschten veränderten mechanischen und optischen Eigenschaften. Diese Problematik ist allgemein unter FRIES Umlagerungen bekannt und wird insbesondere durch die Anwesenheit von Wasser und anderen aus thermischen Abbaureaktionen im BPA gebildete leichtflüchtige Verbindungen begünstigt.

Darüber hinaus können Katalysatorreste zu unerwünschten Nebenreaktionen führen die einen Zerfall bzw. Umsetzung von Rohstoffen bewirken und dadurch das notwendige spezifische molare Verhältnis zwischen der carbonyl- und der hydroxyl-Komopnente verändern. Eine Veränderung des molaren spezifischen Verhältnisses zwischen der Carbonyl- und der Hydroxy-Komponente, kann dazu führen das eine gewünschte Kettenlänge nicht mehr aufgebaut werden kann, aufgrund fehlender funktioneller Endgruppen. Außerdem können bestimmte Verunreinigungen oder die durch Nebenreaktion entstandenen Komponenten dazu führen das das Kettenwachstum gestoppt wird, indem sich diese Komponenten zwar mit der Polymerkette verbinden, aber die für ein weiteres Kettenwachstum benötigten funktionellen Gruppen nicht bereitstellen.

Um ein hochwertiges Polycarbonate herzustellen, ist es daher von größter Bedeutung die erforderlichen Reinheiten der Rohstoffe sicherzustellen.

Aufgrund der großen industriellen Bedeutung von Polycarbonat existieren hierfür zahlreiche Lösungsansätze.

In US7112703 "Production of Bisphenol-A with reduced sulfur content" ist die Trennung von Wasser durch eine Vakuum Destillation und unter anderem durch ein nachgeschaltetes Desorptionsverfahren beschrieben.

In der Patentschrift DE11201300204 "Verfahren zur Herstellung von Bisphenol A" wird ein Herstellungsverfahren von BPA beschrieben indem die flüchtigen Verunreinigungen wie Phenol, Aceton und Wasser durch Destillation abgetrennt werden.

Die Patentschrift US8247619 "BPA and Polycarbonate made from renewable materials" beschreibt die Reinigung von BPA mittels Stripping zur Reduzierung von Verunreinigungen, insbesondere Phenol.

Die Patentschrift US7078573 "Dewatering of circulatory flows in the production of Bisphenol A" beschreibt die Trennung von Wasser von BPA durch eine Destillations Kolonne die unter Vakuum betrieben wird.

In EP0475893 (B1) wird ein Verfahren beschrieben welches mittels fraktionierter Schmelzkristallisation Bisphenol A reinigt um es zur Polycarbonate Herstellung zu verwenden.

In JP5862728 (B2) wird durch die Wahl einer besonderen Hydroxy-Komponente versucht die Empfindlichkeit auf Verunreinigungen zu verringern bzw. die Produkteigenschaften zu verbessern.

In EP1964831 (A1) wird die Herstellung von Diphenylcarbonate und anschließende mehrstufige destillative Reinigung beschrieben.

In WO2014141107(A2) wird beschrieben, dass Katalysatorreste der DPC Herstellung mittels Wasserdampf und anschließender Fällungsreaktion verringert werden.

In DE2439552 wird beschrieben, das ein frühzeitiges Mischen der Hydroxy- und Diaryl-Komponente die Polycarbonat Qualität verbessert.

Die US 8 022 240 B2 beschreibt die destillative Aufreinigung von Diarylcarbonaten. Diese kann nach der Lehre der US 8 022 240 B2 bei geringem apparativen und energetischen Aufwand durchgeführt werden, wenn die zu reinigenden Diarylcarbonate Katalysatorreste und hochsiedende Nebenkomponenten sowie gegebenenfalls auch mittelsiedende Nebenkomponenten als Verunreinigungen enthalten. Dabei wird bevorzugt die bei der Destillation anfallende Abwärme energetisch genutzt.

Nachteilig an den bisherigen Lösungsansätzen ist zum einen die Tatsache, dass die Rohstoffreinigungsanlagen oftmals Bestandteil der Rohstoffherstellungsanlagen sind und daher räumlich getrennt von der Polycarbonat Herstellungsanlage sind. Durch diese räumliche Distanz ergeben sich Verweilzeiten, bedingt durch den Transport von der Rohstoff-Herstellungsanlage zur Polycarbonat-Herstellungsanlage, in denen Verunreinigungen erneut entstehen können und oder neu eingebracht werden können.

Ein weiterer Nachteil ist, dass die etablierten Verfahren die jeweiligen Rohstoffkomponenten separat reinigen, wodurch wiederum Zwischenlagerbehälter erforderlich sind und unvorteilhafte Verweilzeiten entstehen.

Ein weiterer Nachteil ist, dass die etablierten Reinigungssysteme oftmals sehr energieaufwändig sind, da diese einem einmaligen oder mehrmaligen Aggregatszustandswechsel der größten Teilmenge des Rohstoffes erfordern.

Der vorliegenden Erfindung lag die Aufgabe zugrunde, die oben genannten Nachteile der Verfahren nach dem Stand der Technik zu überwinden.

Diese Aufgabe wird erfindungsgemäß gelöst durch ein Verfahren zur Herstellung eines Polycarbonats durch Reaktion eines oder mehrerer Diarylcarbonate mit einem oder mehreren aromatischen Hydroxyverbindungen, wobei Diarylcarbonat und aromatische Hydroxyverbindung zunächst in einer Mischvorrichtung gemischt und als Rohstoffgemisch in einen Rohstoffgemisch-Behälter gelangt um anschließend in einem oder mehreren Reaktoren bei reduziertem Druck und erhöhter Temperatur zu Polycarbonat zu reagieren, wobei das Gemisch aus Diarylcarbonat und aromatischer Hydroxyverbindung vor Eintritt in den Rohstoffgemisch-Behälter eine Strippvorrichtung durchläuft, in der das Rohstoffgemisch im Gegenstromverfahren durch ein Gas gereinigt wird.

Überraschenderweise wurde festgestellt, dass es vorteilhaft ist zur Trennung von Verunreinigungen aus einem Rohstoffgemisch der Polycarbonat-Herstellung ein Gegenstromverfahren mit einer gasförmigen Komponente zu verwenden. Hierdurch kann die Rohstoffqualität, bei verhältnismäßig geringem Aufwand an Energie und Ausrüstung, verbessert werden.

Durch das Vermischen der Rohstoffe ergibt sich ein verbesserter Massentransport und somit ein geringer Ausrüstungsaufwand, als das für den reinen Hydroxyverbindung-Rohstoffstrom der Fall wäre. Darüber hinaus liegt der Erstarrungspunkt der Mischung unterhalb der der aromatischen Hydroxyverbindung-Komponente und ein zusätzlicher Eintrag von Wärme um den Verdampfungsverlust auszugleichen ist nicht notwendig. Zusätzlich stabilisiert die Diarylcarbonat-Komponente die aromtatische Hydroxyverbindung-Komponente.

Erfindungsgemäß wird ein flüssiger Diarylcarbonatstrom, bevorzugt Diphenylcarbonat, mit einem flüssigen aromatischen Hydroxyverbindung-Rohstoffstrom, bevorzugt Bisphenol A, in einer Mischvorrichtung gemischt. Der gemischte Rohstoffstrom wird anschließend in eine Strippvorrichtung gegeben. Gleichzeitig wird in die Strippvorrichtung von unten ein Gasstrom eingeführt, so dass ein Gegenstrom entsteht. Vorzugsweise enthält die Strippvorrichtung spezielle Einbauten, die dazu dienen, den Flüssigkeitsstrom bestmöglich über den Querschnitt des Behälters zu verteilen und die Kontaktfläche von flüssigem Strom und gasförmigen Strom zu erhöhen. Der Druck der Strippvorrichtung wird über eine Regelarmatur in der Gasaustrittsleitung geregelt. Durch den Kontakt der flüssigen und des gasförmigen Stromes, im Gegenstrom, gehen insbesondere die flüchtigen Verunreinigungen in dem gasförmigen Strom über. Der gereinigte Flüssigkeitsstrom wird anschließend mittels gravimetrischer Förderung in einen Rohstoffmischbehälter gefördert, von wo aus er den Reaktoren der Polycarbonat-Produktionsanlage zugeführt wird.

Der Diarylcarbonat-Rohstoffstrom kann direkt aus einer Diarylcarbonat-Herstellungsanlage, mit oder ohne separate Reinigungsanlage, indem der Rohstoffstrom aufbereitet wurde, stammen. Der aromatische Hydroxyverbindung-Rohstoffstrom kann dabei entweder zuvor als Feststoff aufgeschmolzen worden sein mit oder ohne separate Reinigungsanlage oder als Flüssigkeitsstrom direkt aus der Herstellungsanlage mit oder ohne separate Reinigungsanlage, stammen. Die einzelnen Rohstoffströme können in verschiedenen Verhältnissen mittels einer Mischvorrichtung gemischt werden, typischerweise ist das molare Verhältnisse von Diarylcarbonat-Komponente zu Hydroxy-Komponente größer als eins. Denkbar sind allerdings auch Verhältnisse von kleiner als eins oder gleich eins je nachdem was der Prozess erfordert. Es können natürlich noch weitere Komponenten dem Diarylcarbonat-Flüssigkeitsstrom und oder dem aromatischen Hydroxyverbindung-Rohstoffstrom und oder dem gemischten Rohstoffstrom hinzugefügt werden.

Die Strippvorrichtung hat typischerweise ein Verhältnis von Höhe zu Durchmesser von größer als eins. Die Strippvorrichtung ist idealerweise isoliert und beheizt. Die Beheizung kann durch einen Doppelmantel, mit Thermalöl- oder Dampfbeheizung oder durch eine elektrische Begleitbeheizung ausgeführt sein. Die speziellen Einbauten können, als Rohre, als Füllkörper, als Böden oder als Gewebepackungen ausgeführt werden. Idealerweise werden strukturierte Gewbepackungen verwendet. Darüber hinaus können Heizelemente Bestandteil der Einbauten sein.

In einer bevorzugten Ausführungsform ist das erfindungsgemäße Verfahren so ausgestaltet, dass das Reinigen des Rohstoffstroms in der Strippvorrichtung unter verminderten Druck stattfindet, insbesondere unter einem Druck von unter 700 mbar a, bevorzugt unter 600 mbar a, stärker bevorzugt unter einem Druck von unter 100 mbar a und am stärksten bevorzugt unter einem Druck von unter 60 mbar a, insbesondere zwischen 50 mbar a und 10 mbar a.

In einer bevorzugten Ausführungsform ist das erfindungsgemäße Verfahren so ausgestaltet, dass als Gas ein oder mehrere Inertgase verwendet werden

In einer besonders bevorzugten Ausführungsform ist das erfindungsgemäße Verfahren so ausgestaltet, dass als Gas Stickstoff eingesetzt wird.

Allerdings können auch andere inerte Stoffe einzeln oder in Kombination hierfür verwendet werden wie zum Beispiel CO₂, Argon, Helium. Darüber hinaus ist es denkbar den Gasstrom vor Einspeisung in den Behälter zu erwärmen oder zu kühlen. Auch ist es möglich den Gasstrom nach Austritt aus dem Behälter aufzubereiten und zu rezirkulieren. Der gasförmige Strom kann entsprechend der erwarteten Verunreinigung und oder dem Flüssigkeitsstrom bzw. dem Durchsatz der Anlage variiert werden.

In einer bevorzugten Ausführungsform ist das erfindungsgemäße Verfahren so ausgestaltet, dass das Reinigen des Rohstoffstroms in der Strippvorrichtung unter erhöhter Temperatur stattfindet insbesondere über einer Temperatur von 85 °C, bevorzugt über einer Temperatur von über 120 °C, stärker bevorzugt über einer Temperatur von 145°C und am stärksten bevorzugt bei einer Temperatur zwischen 150 °C und 165°C.

In einer bevorzugten Ausführungsform ist das erfindungsgemäße Verfahren so ausgestaltet, dass das Reinigen des Rohstoffstroms in der Strippvorrichtung unter erhöhter Temperatur und verringertem Druck stattfindet insbesondere bei einer Temperatur zwischen 150 °C und 165°C und einem Druck von zwischen 50 mbar a und 10 mbar a.

In einer bevorzugten Ausführungsform ist das erfindungsgemäße Verfahren so ausgestaltet, dass das Verhältnis von flüssigem zu gasförmigen Massenstrom, angegeben in kg/h in der Stripvorrichtung größer als 100 ist, bevorzugt größer als 1000, besonders bevorzugt größer als 2500 und stärker bevorzugt zwischen 3000 und 113000 liegt.

Allerdings sollte dem Fachmann bewusst sein, dass darüber hinaus nicht nur eine Beziehung zwischen Flüssigkeitsstrom und Gasstrom sondern auch zwischen den Verunreinigungen im Flüssigkeitsstrom und dem Gasstrom besteht und daher den Gasstrom beeinflussen kann.

In einer besonders bevorzugten Ausführungsform ist das erfindungsgemäße Verfahren so ausgestaltet, dass die Strippvorrichtung eine Kolonnenzone mit strukturierten Packungen aufweist.

Diese strukturierten Packungen in der Kolonnenzone erhöhen die Kontaktfläche zwischen flüssigen und gasförmigen Strom und erhöhen somit die Effizienz der Aufreinigung.

In einer besonders bevorzugten Ausführungsform ist das erfindungsgemäße Verfahen so ausgestaltet, dass die Strippvorrichtung eine Vorheizzone aufweist, die in Bezug auf die Kolonnenzone in Richtung des Rohstoffgemischstromes stromabwärts liegt.

Eine solche Vorheizzone stromabwärts von der Kolonnenzone hat den Vorteil, dass eine Vorwärmung des Gasstoffstromes erfolgt, bevor der Gasstoffstrom in der Kolonnenzone in intensiven Kontakt mit dem flüssigen Rohstoffstrom tritt. Dadurch dass die Strippvorrichtung typischerweise unter Vakuum betrieben wird und eine starke Entspannung des Gasstromes, typischerweise Stickstoff, gegenüber dem Druck des Gases in der Zuleitung eintritt, kann eine solche Vorheizzone die Effizienz des Reinigungsprozesses deutlich steigern, da eine zu starke Abkühlung des entspannten Gases verhindert wird.

In einer besonders bevorzugten Ausführungsform ist das erfindungsgemäße Verfahren so ausgestaltet, dass die Kolonnenzone der Strippvorrichtung beheizt ist. Auch durch die Beheizung der Kolonnenzone kann die Effizienz des Reinigungsprozesses erhöht werden.

In einer bevorzugten Ausführungsform ist das erfindungsgemäße Verfahren so ausgestaltet, dass die Einleitung des Gases in die Strippvorrichtung über den Rohstoffgemischbehälter erfolgt. Es hat sich gezeigt, dass eine Zuleitung des Gasstromes über den Rohstroffgemischbehälter zu besonders guten Ergebnissen führt, da hierbei bereits ein Kontakt zwischen dem Rohstroffstrom und dem Gasstrom stromabwärts der Strippvorrichtung auftritt, was bereits zu einem gewissen Reinigungseffekt führt und darüber hinaus ein Vorheizen des Gasstromes bewirkt.

In einer bevorzugten Ausführungsform ist das erfindungsgemäße Verfahren so ausgestaltet, dass als aromatische Hydroxyverbindung Dihydroxydiarylalkane der Formel HO-Z-OH verwendet werden, wobei Z ein divalenter organischer Rest mit 6 bis 30 Kohlenstoffatomen ist, der eine oder mehrere aromatische Gruppen enthält.

In einer bevorzugten Ausführungsform ist das erfindungsgemäße Verfahren so ausgestaltet, dass als Diarylcarbonat Di-(C₆ bis C₁₄-Aryl)-Kohlensäureester verwendet werden.

In einer bevorzugten Ausführungsform ist das erfindungsgemäße Verfahren so ausgestaltet, dass dass als aromatische Hydroxyverbindung Bisphenol A und als Diarylcarbonat Diphenylcarbonat verwendet werden.

In einer bevorzugten Ausführungsform ist das erfindungsgemäße Verfahren so ausgestaltet, dass die Mischvorrichtung zum Mischen der Rohstoffströme aus einem so genannten statischen Mischer als Bestandteil einer Rohrleitung besteht.

Darüber hinaus kann in einer besonders bevorzugten Ausführungsform das erfindungsgemäße Verfahren so ausgestaltet sein, dass die Mischvorrichtung zum Mischen der Rohstoffströme aus einem Rohstoffmischbehälter besteht in den die Rohstoffströme kontinuierlich oder batchweise gefördert und vermischt werden.

Ferner wird im Folgenden eine nicht-erfindungsgemäße Strippvorrichtung zur Reinigung eines Rohstoffgemischstromes durch ein Gas im Gegenstromverfahren in einem Verfahren zur Herstellung von Polycarbonat, wobei die Strippvorrichtung eine Kolonnenzone mit strukturierten Packungen aufweist sowie eine Vorheizzone zum Vorheizen des Gases, beschrieben.

Eine solche Strippvorrichtung ist insbesondere zum Einsatz in einem Verfahren zur Herstellung von Polycarbonat einsetzbar.

Die Strippvorrichtung hat typischerweise ein Verhältnis von Höhe zu Durchmesser von größer als eins. Die Strippvorrichtung ist idealerweise isoliert und beheizt. Die Beheizung kann durch einen Doppelmantel, mit Thermalöl- oder Dampfbeheizung oder durch eine elektrische Begleitbeheizung ausgeführt sein. Die speziellen Einbauten können, als Rohre, als Füllkörper, als Böden oder als Gewebepackungen ausgeführt werden. Idealerweise werden strukturierte Gewbepackungen verwendet. Darüber hinaus können Heizelemente Bestandteil der Einbauten sein.

Die Strippvorrichtung kann so ausgeführt sdin, dass sie über Schweißlippendichtungen angeschlossen und vakuumdicht geschlossen werden kann.

Es hat sich gezeigt, dass der Anschluss der Strippvorrichtung über Schweißlippendichtungen gegegenüber den üblicherweise verwendeten Graphitdichtungen zu Vorteilen führt. Dies ist insbesondere der Fall, weil die Strippvorrichtung typischerweise unter Vakuum betrieben wird.

Ferner kann die Strippvorrichtung so ausgeführt sein, dass das Gehäuse und strukturierten Packungen in der Kolonnenzone aus Edelstahl 904L oder höherwertig hergestellt sind. Die Verwendung von Edelstahl insbesondere 904L oder höherwertig führt zur Erhöhung der Langliebigkeit des Strippers und zur Vermeidung von Korrosion und Verminderung von Verfärbungen des Rohstoffs.

Die Ausführung der Erfindung beschränkt sich nicht auf das einmalige Reinigen des flüssigen Rohstoffstroms. Es ist natürlich auch denkbar das Rohstoffgemisch aus dem anschließenden Rohstoffbehälter kontinuierlich oder batchweise über den Behälter zu zirkulieren.

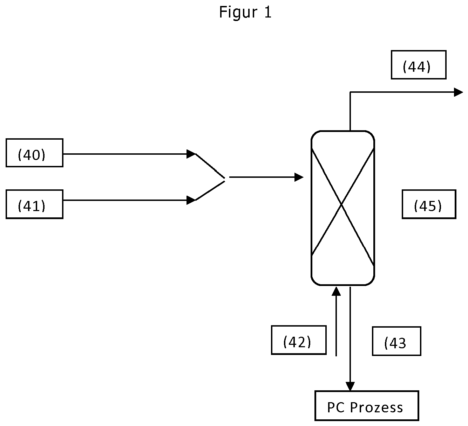

Figur 1 zeigt eine schematische Darstellung des erfindungsgemäßen Verfahrens. Diese schematische Darstellung zeigt das Prinzip der Gegenstromreinigung in einer Kolonnenzone der Stripvorrichtung.

| **Figur 1** | |
|---|---|
| **Nr.** | **Beschreibung** |
| 40 | Diaryl carbonate Komponente |
| 41 | Aromatische Hydroxyl Komponente |
| 42 | Gasförmige Gegenstrom Komponente |
| 43 | Rohstoffstrom |
| 44 | Abgasstrom |
| 45 | Gegenstrom Kolonne |

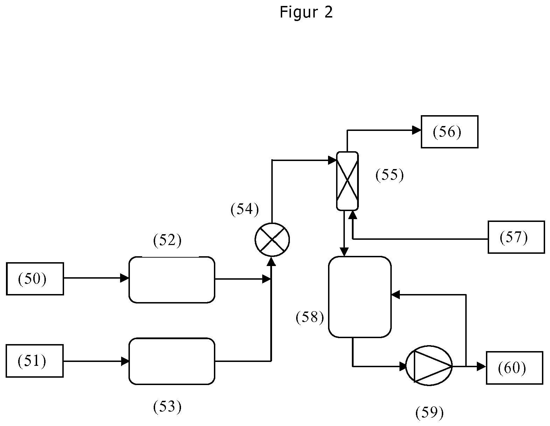

Eine besonders bevorzugte Ausführungsform des erfindungsgemäßen Verfahrens ist in Figur 2 dargestellt.

| **Figur 2** | |
|---|---|
| **Nr.** | **Beschreibung** |
| 50 | Bisphenol A Herstellung |
| 51 | Diphenyl carbonat Herstellung |
| 52 | Bisphenol A Rohstoffbehälter |
| 53 | Diphenyl Carbonat Rohstoffbehälter |
| 54 | Mischvorrichtung |
| 55 | Gegenstrom Kolonne |
| 56 | Vakuumsystem |
| 57 | Stickstoff Versorgung |
| 58 | Rohstoff Mischbehälter |
| 59 | Rohstoffpumpe |
| 60 | Erstes Reaktionssystem |

Die individuellen Rohstoffströme werden aus der jeweiligen Herstellungsanlage (50) und (51) in Vorlage-Behälter (52) und (53) gefördert. Die aus den Vorlage-Behältern geförderten Rohstoffströme werden anschließend in einem bestimmten molaren Verhältnis zusammengeführt und mit einer Mischvorrichtung (54) durchmischt. Das Rohstoffgemisch wird dann in die Strippvorrichtung (55) eingespeist. Gleichzeitig wird im Gegenstrom Stickstoff aus der Stickstoff Versorgung (57) von unten in die Strippvorrichtung(55) eingespeist. Durch den Kontakt mit dem flüssigen Gegenstrom reichern sich Verunreinigungen im Stickstoffstrom an. Der angereicherte Gasstrom verlässt die Strippvorrichtung am Kopf und wird zum Vakuumsystem weitergeleitet (56). Der gereinigte Rohstoffstrom tritt am Boden der Strippvorrichtung (55) aus in einen Rohstoff-Mischbehälter (58). Mittels Rohstoffpumpe (59) wird das Rohstoffgemisch zum ersten Reaktionssystem (60) gefördert.

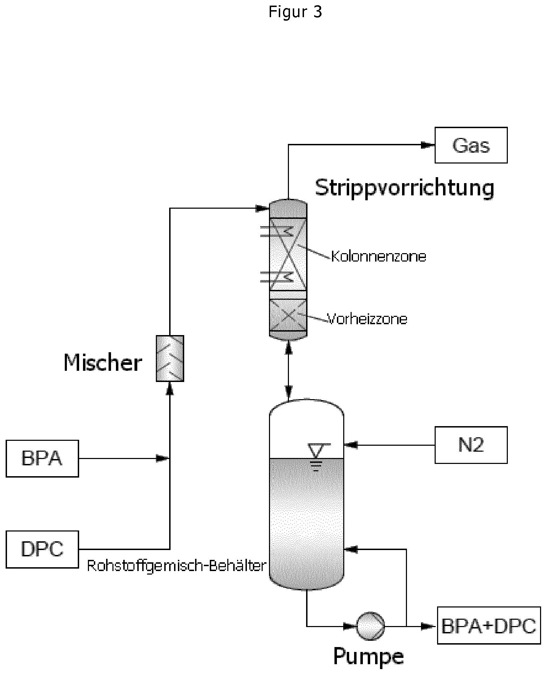

Figur 3 zeigt eine besonders bevorzugte Ausführungsform des erfindungsgemäßen Verfahrens, bei der die Strippvorrichtung eine Vorheizzone aufweist und die Einleitung des Gases in Form von Stickstoff über den Rohstoffgemischbehälter erfolgt.

## Patentansprüche

1. Verfahren zur Herstellung eines Polycarbonats durch Reaktion eines oder mehrerer Diarylcarbonate mit einem oder mehreren aromatischen Hydroxyverbindungen, wobei Diarylcarbonat und aromatische Hydroxyverbindung zunächst in einer Mischvorrichtung gemischt und als Rohstoffgemisch in einen Rohstoffgemisch-Behälter gelangt um anschließend in einem oder mehreren Reaktoren bei reduziertem Druck und erhöhter Temperatur zu Polycarbonat zu reagieren, wobei das Gemisch aus Diarylcarbonat und aromatischer Hydroxyverbindung vor Eintritt in den Rohstoffgemisch-Behälter eine Strippvorrichtung durchläuft, in der das Rohstoffgemisch im Gegenstromverfahren durch ein Gas gereinigt wird.

2. Verfahren gemäß Anspruch 1,
**dadurch gekennzeichnet, dass**
das Reinigen des Rohstoffstromes in der Strippvorrichtung unter verminderten Druck stattfindet, insbesondere unter einem Druck von unter 0.700 bar a, stärker bevorzugt unter einem Druck von unter 0.600 bar a, besonders bevorzugt unter einem Druck von unter 0.100 bar a und am stärksten bevorzugt unter einem Druck von unter 0.060 bar a insbesondere zwischen 0.050 bar a und 0.010 bar a.

3. Verfahren nach Anspruch 1 oder 2,
**dadurch gekennzeichnet, dass**
als Gas ein oder mehrere Inertgase verwendet werden.

4. Verfahren nach einem der vorhergehenden Ansprüche,
**dadurch gekennzeichnet, dass**
als Gas Stickstoff eingesetzt wird.

5. Verfahren nach einem der vorhergehenden Ansprüche,
**dadurch gekennzeichnet, dass**
das Verhältnis von flüssigem zu gasförmigen Massenstrom, angegeben in kg/h in der Stripvorrichtung größer als 100 ist, bevorzugt größer als 1000, besonders bevorzugt größer als 2500 und stärker bevorzugt zwischen 3000 und 113000 liegt.

6. Verfahren nach mindestens einem der vorhergehenden Ansprüche,
**dadurch gekennzeichnet, dass**
die Strippvorrichtung eine Kolonnenzone mit strukturierten Packungen aufweist.

7. Verfahren nach mindestens einem der vorhergehenden Ansprüche,
**dadurch gekennzeichnet, dass**
die Strippvorrichtung eine Vorheizzone aufweist, die in Bezug auf die Kolonnenzone in Richtung des Rohstoffgemischstromes stromabwärts liegt.

8. Verfahren nach mindestens einem der vorhergehenden Ansprüche,
**dadurch gekennzeichnet, dass**
die Kolonnenzone der Strippvorrichtung beheizt ist.

9. Verfahren nach mindestens einem der vorhergehenden Ansprüche,
**dadurch gekennzeichnet, dass**
die Einleitung des Gases in die Strippvorrichtung über den Rohstoffgemischbehälter erfolgt.

10. Verfahren nach mindestens einem der vorhergehenden Ansprüche,
**dadurch gekennzeichnet, dass**
als aromatische Hydroxyverbindung Dihydroxydiarylalkane der Formel HO-Z-OH verwendet werden, wobei Z ein divalenter organischer Rest mit 6 bis 30 Kohlenstoffatomen ist, der eine oder mehrere aromatische Gruppen enthält.

11. Verfahren nach mindestens einem der vorhergehenden Ansprüche,
**dadurch gekennzeichnet, dass**
als Diarylcarbonat Di-(C₆ bis C₁₄-Aryl)-Kohlensäureester verwendet werden.

12. Verfahren nach mindestens einem der vorhergehenden Ansprüche,
**dadurch gekennzeichnet, dass**
dass als aromatische Hydroxyverbindung Bisphenol A und als Diarylcarbonat Diphenylcarbonat verwendet werden.

## Claims

1. A process for producing a polycarbonate by reaction of one or more diaryl carbonates with one or more aromatic hydroxy compounds, wherein diaryl carbonate and aromatic hydroxy compound are first mixed in a mixing device and enter, as a raw material mixture, a raw material mixture container to subsequently react in one or more reactors under reduced pressure and increased temperature to form polycarbonate, wherein the mixture of diaryl carbonate and aromatic hydroxy compound passes through a stripping device in which the raw material mixture is purified by a gas in a countercurrent process before entering the raw material mixture container.

2. The process according to claim 1, **characterized in that** the purification of the raw material stream in the stripping device takes place under reduced pressure, in particular under a pressure of less than 0.700 bar a, more preferably under a pressure of less than 0.600 bar a, still more preferably under a pressure of less than 0.100 bar a, and most preferably under a pressure of less than 0.060 bar a, in particular between 0.050 bar a and 0.010 bar a.

3. The process according to claim 1 or 2, **characterized in that** one or more inert gases are used as the gas.

4. The process according to one of the preceding claims, **characterized in that** nitrogen is used as the gas.

5. The process according to one of the preceding claims, **characterized in that** the ratio of liquid to gaseous mass flow, expressed in kg/h, in the stripping device is greater than 100, preferably greater than 1000, more preferably greater than 2500 and more preferably between 3000 and 113000.

6. The process according to at least one of the preceding claims, **characterized in that** the stripping device has a column zone with structured packings.

7. The process according to at least one of the preceding claims, **characterized in that** the stripping device has a preheating zone, which is located downstream with respect to the column zone in the direction of the raw material mixture stream.

8. The process according to at least one of the preceding claims, **characterized in that** the column zone of the stripping device is heated.

9. The process according to at least one of the preceding claims, **characterized in that** the gas is introduced into the stripping device via the raw material mixture container.

10. The process according to at least one of the preceding claims, **characterized in that** dihydroxydiarylalkanes of the formula HO-Z-OH, wherein Z is a divalent organic residue having 6 to 30 carbon atoms, which contains one or more aromatic groups, are used as the aromatic hydroxy compound.

11. The process according to at least one of the preceding claims, **characterized in that** di-(C₆ to C₁₄ aryl) carbonic acid esters are used as the diaryl carbonate.

12. The process according to at least one of the preceding claims, **characterized in that** bisphenol A is used as aromatic hydroxy compound and diphenyl carbonate is used as diaryl carbonate.

## Revendications

1. Procédé de fabrication d'un polycarbonate par réaction d'un ou de plusieurs carbonates de diaryle avec un ou plusieurs composés hydroxy aromatiques, dans lequel le carbonate de diaryle et le composé hydroxy aromatique sont mélangés dans un premier temps dans un dispositif de mélange et parviennent en tant que mélange de matières premières dans un contenant de mélange de matières premières pour réagir ensuite en polycarbonate dans un ou plusieurs réacteurs à une pression réduite et à une température augmentée, dans lequel le mélange de carbonate de diaryle et d'un composé hydroxy aromatique traverse avant d'entrer dans le contenant de mélange de matières premières un dispositif de strippage, dans lequel le mélange de matières premières est purifié par un gaz dans le procédé à contre-courant.

2. Procédé selon la revendication 1,
**caractérisé en ce que**
le nettoyage du flux de matières premières dans le dispositif de strippage a lieu sous une pression réduite, en particulier sous une pression inférieure à 0,700 bar a, de manière davantage préférée sous une pression inférieure à 0,600 bar a, de manière particulièrement préférée sous une pression inférieure à 0,100 bar a et idéalement sous une pression inférieure à 0,060 bar a en particulier entre 0,050 bar a et 0,010 bar a.

3. Procédé selon la revendication 1 ou 2,
**caractérisé en ce qu'**un ou plusieurs gaz inertes sont utilisés en tant que gaz.

4. Procédé selon l'une quelconque des revendications précédentes,
**caractérisé en ce que**
de l'azote est employé en tant que gaz.

5. Procédé selon l'une quelconque des revendications précédentes,
**caractérisé en ce que**
le rapport du flux massique fluide par rapport au flux massique gazeux indiqué en kg/h dans le dispositif de strippage est supérieur à 100, de manière préférée est supérieur à 1000, de manière particulièrement préférée est supérieur à 2500 et de manière davantage préférée est compris entre 3000 et 113 000.

6. Procédé selon au moins l'une quelconque des revendications précédentes,
**caractérisé en ce que**
le dispositif de strippage présente une zone de colonnes avec des enveloppements structurés.

7. Procédé selon au moins l'une quelconque des revendications précédentes,
**caractérisé en ce que**
le dispositif de strippage présente une zone de préchauffage, qui se situe en aval en direction du flux de mélange de matières premières par rapport à la zone de colonnes.

8. Procédé selon au moins l'une quelconque des revendications précédentes,
**caractérisé en ce que**
la zone de colonne du dispositif de strippage est chauffée.

9. Procédé selon au moins l'une quelconque des revendications précédentes,
**caractérisé en ce que**
l'introduction du gaz dans le dispositif de strippage est effectuée par l'intermédiaire du contenant de mélange de matières premières.

10. Procédé selon au moins l'une quelconque des revendications précédentes,
**caractérisé en ce que**
des dihydroxydiarylalcanes de la formule HO-Z-OH sont utilisés en tant que composé hydroxy aromatique, dans lequel Z est un radical organique divalent avec 6 à 30 atomes de carbone, qui contient un ou plusieurs groupes aromatiques.

11. Procédé selon au moins l'une quelconque des revendications précédentes,
**caractérisé en ce que**
des esters d'acide dicarboxylique (d'aryle en Ce à C₁₄) sont utilisés en tant que carbonate de diaryle.

12. Procédé selon au moins l'une des revendications précédentes,
**caractérisé en ce que**
du bisphénol A est utilisé en tant que composé hydroxy aromatique et du carbonate de diphényle est utilisé en tant que carbonate de diaryle.
